(19) Europäisches Patentamt
European Patent Office
Office européen des brevets

(11) Veröffentlichungsnummer: **0 084 783**
**A1**

# (12) EUROPÄISCHE PATENTANMELDUNG

(21) Anmeldenummer: **83100109.4**

(22) Anmeldetag: **08.01.83**

(51) Int. Cl.[3]: **C 07 C 119/12**

(30) Priorität: **23.01.82 DE 3202056**

(43) Veröffentlichungstag der Anmeldung: **03.08.83**
**Patentblatt 83/31**

(84) Benannte Vertragsstaaten: **BE DE FR GB IT NL**

(71) Anmelder: **BAYER AG, Zentralbereich Patente, Marken und Lizenzen, D-5090 Leverkusen 1, Bayerwerk (DE)**

(72) Erfinder: **Hajek, Manfred, Dr., Hahnenweg 1, D-5000 Köln 80 (DE)**

(54) **Verfahren zur Herstellung von N-Isopropylidenaminen.**

(57) Ein Verfahren zur Herstellung von N-Isopropylidenaminen durch Umsetzung von aliphatischen, cycloaliphatischen oder araliphatischen primären Aminen mit Mesityloxyd und/oder Diacetonalkohol unter gleichzeitiger Abspaltung von Wasser.

EP 0 084 783 A1

ACTORUM AG

BAYER AKTIENGESELLSCHAFT 5090 Leverkusen, Bayerwerk

Zentralbereich
Patente, Marken und Lizenzen Wr-by-c

## Verfahren zur Herstellung von N-Isopropylidenaminen

Die vorliegende Erfindung betrifft ein neues Verfahren zur Herstellung von Ketiminen des Acetons (N-Propylidenaminen) durch Umsetzung von bestimmten primären Aminen mit Mesityloxyd oder Diacetonalkohol.

Ketimine stellt man nach literaturbekannten Methoden her, wie sie z.B. in Chem. Rev. 63, 489 ff beschrieben sind. Das wichtigste Verfahren beruht auf der Kondensation eines Ketons mit einem primären Amin unter Wasserabspaltung. Um das Reaktionsgleichgewicht auf die Seite des Ketimins zu verschieben, ist es notwendig, das bei der Reaktion entstehende Wasser zu entfernen. Das technisch interessanteste Verfahren zur Entfernung des Wassers besteht hierbei in einer azeotropen Destillation unter Mitverwendung eines Schleppmittels. Gerade dieses Verfahren ist jedoch bei der Herstellung der technisch besonders interessanten Ketimine des Acetons wegen dessen niedrigen Siedepunkts und seiner Mischbarkeit mit Wasser schwierig zu handhaben bzw. unmöglich, da in den meisten Fällen kein geeignetes azeotropes Gemisch gebildet wird. Aus diesem

Grund wird in der Literatur D.G. Morton et. al. J. Org. Chem. 19, 1054 (1954)) eine Entfernung des Reaktionswassers beispielsweise durch Zusatz von Natriumhydroxid empfohlen.

Überraschenderweise wurde nunmehr ein neuartiger Weg zu 2-Propylidenaminen gefunden, der darin besteht, daß man geeignete primäre Amine mit Mesityloxyd oder Diacetonalkohol umsetzt und nach üblichen Methoden das Wasser azeotrop aus dem Reaktionsgemisch entfernt. Die Durchführbarkeit dieses, nachstehend näher beschriebenen, erfindungsgemäßen Verfahrens ist überraschend, da es bekannt war, daß z.B. bei der Reaktion zwischen Ethylendiamin und Mesityloxyd nicht das entsprechende Bisketimin des Acetons entsteht (J.Guareschi, Ber. 28, Ref. 161 (1895).

Gegenstand der Erfindung ist ein Verfahren zur Herstellung von N-Isopropylidenaminen der allgemeinen Formel

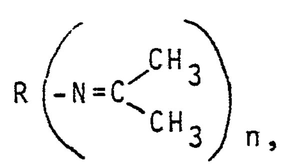

in welcher

n    für eine ganze Zahl von 1 bis 3 steht und

R für einen n-wertigen aliphatischen Kohlenwasserstoffrest mit 1 bis 18 Kohlenstoffatomen, cycloaliphatischen Kohlenwasserstoffrest mit 4 bis 15 Kohlenwasserstoffatomen oder araliphatischen Kohlenwasserstoffrest mit 7 bis 15 Kohlenstoffatomen steht, wobei im Falle von n = 2 oder 3 zwischen den, mit R verknüpften, Stickstoffatomen mindestens 3 Kohlenstoffatome des Restes R angeordnet sind,

dadurch gekennzeichnet, daß man Amine der allgemeinen Formel

$$R(-NH_2)_n$$

mit Mesityloxyd oder Diacetonalkohol unter Wasserabspaltung umsetzt.

Für das erfindungsgemäße Verfahren eignen sich beliebige Amine der obengenannten allgemeinen Formel, wobei n und R die bereits obengenannte Bedeutung haben. Besonders bevorzugt werden Diamine (n = 2) der genannten allgemeinen Formel eingesetzt, für welche

R für einen gesättigten aliphatischen Kohlenwasserstoffrest mit 4 bis 10 Kohlenstoffatomen, insbesondere einen Hexamethylenrest, einen gesättigten cycloaliphatischen oder aliphatisch-cycloaliphatischen Kohlenwasserstoffrest mit 6 bis 15 Kohlenstoffatomen oder einen Xylylenrest steht, wobei unter "aliphatisch-cycloaliphatischen" Resten solche zu verstehen sind, die neben mindestens einer cycloaliphatischen Struktureinheit Alkylen- insbesondere Methylenbrücken und/oder Alkyl- insbesondere Methylsubstituenten aufweisen, wobei die freien, mit dem Aminstickstoff verbundenen Valenzen sowohl mit aliphatisch gebundenen als auch mit cycloaliphatisch gebundenen Kohlenstoffatomen verknüpft sein können.

Wie bereits oben dargelegt, sind in derartigen Diamin-Ausgangsmaterialien zwischen den beiden Aminogruppen mindestens 3 Kohlenstoffatome des Kohlenwasserstoffrests angeordnet.

Als Amine eignen sich für das erfindungsgemäße Verfahren beispielsweise:
Methylamin, Ethylamin, Propylamin, Isopropylamin, Butylamin, sec.-Butylamin, Isobutylamin, t-Butylamin, Pentylamin, Hexylamin, 2-Ethylhexylamin, Dodecylamin, Tetradecylamin, Hexadecylamin, Octodecylamin, Cyclohexylamin, Aminomethylcyclohexan, 1,3-Diaminopropan, 1,4-Diaminobutan, 1,6-Diaminohexan, 2,5-Dimethyl-2,5-hexandiamin, 2,2,4- und 2,4,4-Trimethyl-1,6-hexandiamin, Benzylamin, 1,3-Diamino-4-methyl-cyclohexan, 1,4-Diaminocyclohexan, 5-Amino-1-aminomethyl-1,3,3-trimethylcyclohexan (Isophorondiamin), 4,4'-Methylenbiscyclohexylamin, Bis-(aminomethyl)-tricyclodecan, 1,6,11-Triaminoundecan.

Außer diesen beispielhaft genannten Aminen werden beim erfindungsgemäßen Verfahren Mesityloxyd und/oder Diacetonalkohol als Ausgangsmaterial eingesetzt.

Die Mengenverhältnisse der Reaktionspartner werden dabei so gewählt, daß auf jedes Aminäquivalent 0,5 bis 1 Mol, vorzugsweise 0,5 bis 0,7 Mol Mesityloxyd oder Diacetonalkohol entfallen.

Das erfindungsgemäße Verfahren wird im allgemeinen im Temperaturbereich von 10 bis 200°C, vorzugsweise 20 bis 120°C durchgeführt.

Das erfindungsgemäße Verfahren kann in Gegenwart oder in Abwesenheit von Lösungsmitteln durchgeführt werden. Geeignete Lösungsmittel sind insbesondere inerte organische Flüssigkeiten, die mit Wasser nicht mischbar sind, wie z.B. Cyclohexan, Benzol, Toluol oder Xylol. Diese Lösungsmittel können dann gleichzeitig die Funktion eines Schleppmittels für das entstandene Wasser übernehmen. So kann man beispielsweise die erfindungsgemäße Umsetzung dergestalt durchführen, daß man eine Mischung der Ausgangskomponenten in einem inerten Lösungsmittel unter Rückfluß an einem Wasserabscheider (z.B. nach Stark-Dean) kocht und das im Laufe der Reaktion entstehende Wasser abtrennt.

Es kann auch vorteilhaft sein, die Reaktionskomponenten zu mischen und erst nach einer gewissen Zeit mit dem Lösungs- bzw. Schleppmittel zu versetzen.

Oftmals empfiehlt sich bei der Durchführung des erfindungsgemäßen Verfahrens ein Arbeiten unter Inertgasatmosphäre (Stickstoff). Das Ende der erfindungsgemäßen Umsetzung kann an der Beendigung der Wasserbildung erkannt werden. Die erfindungsgemäß erhaltenen Reaktionsgemische können in an sich bekannter Weise, insbesondere durch Destillation aufgearbeitet werden.

Das erfindungsgemäße Verfahren ermöglicht die Herstellung von 2-Propylidenaminen nach einem technisch besonders leicht durchführbaren Verfahren. Die erfindungsgemäßen, mindestens 2 Ketimingruppen aufweisenden Verfahrensprodukte stellen reversibel blockierte Polyamine dar, die beispielsweise in Kombination mit, gegebenenfalls blockierten, Polyisocyanaten, insbesondere NCO-Präpolymeren zu feuchtigkeithärtbaren Systemen (beispielsweise Dichtungsmassen) verarbeitet werden können. Die erfindungsgemäßen, eine Ketimingruppe aufweisenden Verfahrensprodukte stellen interessante Zwischenprodukte für organische Synthesen dar.

In den nachfolgenden Beispielen beziehen sich alle Prozentangaben auf Gewichtsprozente.

0084783

Beispiel 1

In einer 1-ltr.-Dreihalskolbenapparatur (Rührer, Kühler und Wasserabscheider, Gaseinleitungsrohr) mischt man 98 g (1,0 Mol) 4-Methyl-3-penten-2-on (Mesityloxyd) mit 146 g (2,0 Mol) 2-Methyl-1-propanamin. Nach 24 Stunden bei Raumtemperatur setzt man 300 ml Cyclohexan zu und erwärmt unter Stickstoff langsam bis zur Siedetemperatur des Reaktionsgemisches. Anschließend läßt man unter Rückfluß am Wasserabscheider kochen, bis sich kein Wasser mehr abtrennt.

Durch fraktionierte Destillation erhält man zwischen 118 bis 123°C bei Normaldruck 143 g (≙ 63 % d. Th.) N-Isopropyliden-2-methyl-1-propanamin.

Beispiel 2

Verfährt man nach Beispiel 1, nur daß man statt Mesityloxyd 116 g (1,0 Mol) 4-Hydroxy-4-methyl-2-pentanon (Diacetonalkohol) einsetzt, so erhält man 95 g (≙ 42 % d. Th.) N-Isopropyliden-2-methyl-1-propanamin.

Beispiel 3

In einer Apparatur, wie sie im Beispiel 1 benutzt wird, gibt man zu 198 g (2,0 Mol) Cyclohexylamin und 300 ml Toluol innerhalb von 20 Minuten 116 g (1,0 Mol) Diacetonalkohol. Anschließend erwärmt man das Reaktionsgemisch

am Wasserabscheider, bis die Wasserabspaltung beendet ist (ca. 18 Stunden).

Durch fraktionierte Destillation erhält man zwischen 179 und 183°C bei Normaldruck 132 g (≙ 47 % d. Th.) N-Isopropyliden-cyclohexylamin.

Beispiel 4

Man verfährt, wie im Beispiel 3 beschrieben, nur mischt man 198 g (2,0 Mol) Cyclohexylamin und 300 ml Cyclohexan mit 98 g (1,0 Mol) Mesityloxid und läßt diesen Ansatz 36 Stunden bei Raumtemperatur stehen. Anschließend verfährt man wie beschrieben und fraktioniert nach beendeter Wasserabspaltung. Man erhält 193 g (≙ 69 % d. Th.) N-Isopropyliden-cyclohexylamin, das bei Normaldruck zwischen 178 und 184°C überdestilliert.

Beispiel 5

In einer Apparatur, wie sie im Beispiel 1 benutzt wird, mischt man 107 g (1,0 Mol) Benzylamin mit 49 g (0,5 Mol) Mesityloxyd und läßt 18 Stunden bei Raumtemperatur stehen. Anschließend verdünnt man mit 300 ml Cyclohexan und kreist das entstandene Wasser durch Kochen unter Rückfluß am Wasserabscheider aus.

Anschließend wird das Schleppmittel unter vermindertem Druck abdestilliert. Man erhält 160 g rohes Ketimin, das

nach gaschromatographischer Analyse 61 % N-Isopropyliden-benzylamin enthält (≙ 66 % d. Th.).

Beispiel 6

Man verfährt wie im vorausgegangenem Beispiel, nur setzt man als Carbonylverbindung 58 g (0,5 Mol) Diacetonalkohol ein und verfährt wie beschrieben.

Durch fraktionierte Destillation erhält man 79 g (≙ 61 % d. Th.) N-Isopropyliden-benzylamin vom Siedebereich 60-65°C bei 0,2 bis 0,3 mbar.

Beispiel 7

In einer Apparatur, wie sie im Beispiel 1 benutzt wurde, mischt man 98 g (1,0 Mol) Mesityloxyd und 116 g (1,0 Mol) Hexamethylendiamin und läßt 36 Stunden bei Raumtemperatur stehen. Anschließend verdünnt man mit 300 ml Toluol und läßt unter Rückfluß am Wasserabscheider kochen, bis sich kein Wasser mehr auskreisen läßt. Am Rotationsverdampfer destilliert man bei vermindertem Druck alle flüchtigen Bestandteile ab (Badtemperatur max. 80°). Man erhält 208 g rohes Bisketimin, das nach gaschromatographischer Analyse 63 % N,N'-Bisisopropyliden-1,6-diaminohexan enthält. Die berechnete Ausbeute beträgt 66 % der Theorie.

Beispiel 8

In einer Apparatur, wie sie im Beispiel 1 benutzt wird, erwärmt man eine Mischung aus 116 g (1,0 Mol) Diacetonalkohol, 116 g (1 Mol) Hexamethylendiamin und 300 ml Toluol 2 Stunden auf 60°C und steigert anschließend die Temperatur, bis das Reaktionsgemisch unter Rückfluß am Wasserabscheider kocht. Nach etwa 8 Stunden ist die Wasserabscheidung beendet. Man destilliert alle niedrigsiedenden Anteile ab und unterwirft das rohe Bisketimin einer fraktionierten Destillation. Man erhält bei einem Druck von 0,1 bis 0,2 mbar und einer Temperatur von 74 bis 80°C 110 g (= 56 % d.Th.) Bis-isopropyliden-1,6-diaminohexan.

Beispiel 9

In einer Apparatur, wie im Beispiel 1 beschrieben, stellt man eine Mischung aus 170 g (1,0 Mol) Isophorondiamin und 98 g (1,0 Mol) Mesityloxyd her und läßt diese 48 Stunden bei Raumtemperatur stehen. Anschließend verdünnt man mit 300 ml Toluol und erwärmt langsam bis zur Rückflußtemperatur.

Nach etwa 14 Stunden ist die Wasserabscheidung beendet, und die Reaktion wird abgebrochen. Die niedrigsiedenden Bestandteile werden am Rotationsverdampfer bei Wasserstrahlpumpenvakuum abgetrennt. Man erhält 252 g Rohprodukt, das nach fraktionierter Destillation bei 0,15 mbar 193 g (≙ 77 % d. Th.) Bisketimin liefert ($Kp._{0,15}$: 98-103°C).

Beispiel 10

In einer Apparatur, wie im Beispiel 1 beschrieben, läßt man eine Mischung aus 170 g (1,0 Mol) Isophorondiamin und 98 g (1,0 Mol) Mesityloxyd 18 Stunden bei Raumtemperatur stehen. Nach Zugabe von 300 ml Cyclohexan bringt man die Mischung zum Sieden und trennt das entstehende Wasser durch azeotrope Destillation ab. Nach etwa 6 Stunden ist die Reaktion beendet, und man entfernt im Vakuum alle flüchtigen Bestandteile am Rotationsverdampfer. Man erhält 258 g rohes Bisketimin, das nach fraktionierter Destillation 170 g Bis-isopropyliden-isophorondiamin liefert ($Kp._{0,15}$: 98 bis 102°C). Die Ausbeute beträgt 68 % d. Th.

Beispiel 11

In einer Apparatur, wie im Beispiel 1 beschrieben, bringt man nach einer Vorreaktion von 48 Stunden bei Raumtemperatur eine Mischung aus 170 g (1,0 Mol) Isophorondiamin, 300 ml Cyclohexan und 108 g (1,0 Mol) Mesityloxyd zum Sieden und kreist innerhalb von 18 Stunden das entstehende Wasser aus. Man arbeitet wie im Beispiel 10 beschrieben auf.

Man erhält 148 g (= 59 % d. Zh.) N,N'-Bis-isopropyliden-isophorondiamin ($Kp._{0,15}$: 95-102°C).

Beispiel 12

Man verfährt wie im Beispiel 11 angegeben, nur setzt man 123 g (1,25 Mol) Mesityloxyd ein.

Man erhält 276 g Rohprodukt, das nach Fraktionierung 180 g (= 72 % d. Th.) N,N'-Bis-isopropyliden-isophorondiamin liefert.

Beispiel 13

Man verfährt wie im Beispiel 11, nur setzt man 196 g (2,0 Mol) Mesityloxyd ein und arbeitet wie im gleichen Beispiel beschrieben auf.

Man erhält 130 g N,N'-Bis-isopropyliden-isophorondiamin (= 52 % d. Th.).

Beispiel 14

Man verfährt wie im Beispiel 9 beschrieben, nur setzt man statt des Mesityloxids 116 g (1,0 Mol) Diacetonalkohol ein. Nach Aufarbeitung erhält man 270 g Rohprodukt das nach Fraktionierung 148 g (= 60 % d. Th.) N,N'-Bis-isopropyliden-isophorondiamin liefert.

Patentansprüche

1. Verfahren zur Herstellung von N-Isopropylidenaminen der allgemeinen Formel

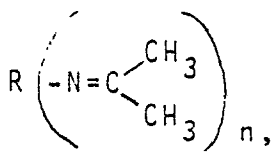

in welcher

n für eine ganze Zahl von 1 bis 3 steht und

R für einen n-wertigen aliphatischen Kohlenwasserstoffrest mit 1 bis 18 Kohlenstoffatomen, cycloaliphatischen Kohlenwasserstoffrest mit 4 bis 15 Kohlenstoffatomen oder araliphatischen Kohlenwasserstoffrest mit 7 bis 15 Kohlenstoffatomen steht, wobei im Falle von n = 2 oder 3 zwischen den, mit R verknüpften, Stickstoffatomen mindestens 3 Kohlenstoffatome des Restes R angeordnet sind,

dadurch gekennzeichnet, daß man Amine der allgemeinen Formel

$$R(-NH_2)_n$$

mit Mesityloxyd oder Diacetonalkohol unter Wasserabspaltung umsetzt.

2. Verfahren nach Anspruch 1, dadurch gekennzeichnet, daß man pro Äquivalent Amin 0,5 Mol bis 1 Mol Mesityloxyd oder Diacetonalkohol einsetzt.

3. Verfahren nach Anspruch 1 und 2, dadurch gekennzeichnet, daß man das Reaktionswasser durch azeotrope Destillation unter Verwendung eines, unter Normaldruck zwischen 40°C und 200°C siedenden, Schleppmittels aus dem Reaktionsgemisch entfernt.

Europäisches Patentamt

# EUROPÄISCHER RECHERCHENBERICHT

0084783
Nummer der Anmeldung

EP 83 10 0109

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-4 306 068 (SMITH)<br>* Spalte 2 *<br>----- | 1-3 | C 07 C 119/12 |
| | | | RECHERCHIERTE SACHGEBIETE (Int. Cl. 3) |
| | | | C 07 C 119/00 |

Der vorliegende Recherchenbericht wurde für alle Patentansprüche erstellt.

| Recherchenort | Abschlußdatum der Recherche | Prüfer |
|---|---|---|
| DEN HAAG | 27-04-1983 | GAUTIER R.H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82